# EUROPEAN PATENT SPECIFICATION

(11) **EP 3 476 229 B1**
(45) Date of publication and mention of the grant of the patent: **06.04.2022**
(21) Application number: 16907227.9
(22) Date of filing: 27.06.2016
(51) Int. Cl.: A24F 40/42, A24F 40/46, A24F 40/10

(54) **CARTRIDGE FOR AEROSOL INHALER, AEROSOL INHALER PROVIDED WITH SAME, AND HEAT-GENERATING SHEET FOR AEROSOL INHALER**
KARTUSCHE FÜR AEROSOLINHALATOR, AEROSOLINHALATOR DAMIT UND WÄRMEERZEUGENDE SCHICHT FÜR AEROSOLINHALATOR
CARTOUCHE POUR INHALATEUR D'AÉROSOL, INHALATEUR D'AÉROSOLMUNI DE CELLE-CI ET PLAQUE PRODUCTRICE DE CHALEUR POUR INHALATEUR D'AÉROSOL

(43) Date of publication of application: 01.05.2019
(73) Proprietor: Japan Tobacco Inc., Tokyo 105-8422 (JP)
(72) Inventor: MATSUMOTO, Hirofumi, Tokyo 130-8603 (JP); NAKANO, Takuma, Tokyo 130-8603 (JP); YAMADA, Manabu, Tokyo 130-8603 (JP); OISHI, Kei, Tokyo 130-8603 (JP)
(74) Representative: Hoffmann Eitle
(86) International application number: PCT/JP2016/069033
(87) International publication number: WO 2018/002994

(56) References cited:
- WO-A1-2015/114327
- WO-A1-2016/092261
- JP-A- 2008 545 943
- JP-A- 2012 506 263
- JP-A- 2014 525 237
- JP-A- 2015 504 652
- JP-A- 2015 506 182
- US-A- 4 588 976
- US-A1- 2013 081 623

## Description

### Technical Field

The present invention relates to a cartridge for aerosol inhaler, an aerosol inhaler provided with the same, and a heat-generating sheet for aerosol inhaler.

### Background Art

An aerosol inhaler has been known which generates aerosols following a user's inhalation action and provides the aerosols to the user. One example of this type of an aerosol inhaler is an electronic cigarette in which an aerosol-generating liquid is atomized (aerosolized) in an atomizing unit through electric heating using an electric heating coil or the like. The aerosol-generating liquid is a liquid for generating aerosols, and examples thereof include glycerol (G) and propylene glycol (PG).

For example, the aerosol-generating liquid is impregnated in and held by a reservoir made of cotton or the like, a wick made of glass fibers or the like suctions the aerosol-generating liquid from the reservoir by using the capillary effect, and the aerosol-generating liquid is transmitted to the vicinity of the electric heating coil. Furthermore, the electric heating coil is commonly made of a nichrome wire or the like, and is wound around the wick made of glass fibers. However, in such a mode, a resistance value changes according to the wound state of the electric heating coil around the wick. This easily generates quality variation so that the cost for inspections, etc. are increased in some cases.

In this respect, a technology for providing a liquid absorbing capacity by using a porous material for a heater material itself of an atomizing unit, has been proposed. For example, JP 5612585 B discloses a technology related to an aerosol inhaler that adopts, for a heater having a capillary structure, a woven fabric structure, a fiber structure having open holes, a sintered structure having open holes, a foam having open holes, a precipitation structure having open holes, or the like.

WO 2016/092261 A1 is related to a cartridge for an aerosol inhaler having a liquid reservoir and a heating element provided with a positive and a negative electrode and adapted to atomize the aerosol generating liquid supplied from the liquid reservoir by generating heat when a current flow is caused between the electrodes. The heating element is porous, and a meandering electric path is formed by means of a slit.

### Summary of Invention

### Technical Problem

However, in the technology disclosed in Patent document 1, there is room for improvement in the heat generating properties demanded of the heater which is used for the aerosol inhaler.

The present invention has been made in view of the aforementioned circumstances, and an object thereof is to provide a technology of a heat-generating sheet for aerosol inhaler for use in atomizing an aerosol-generating liquid such that a resistance value demanded of a heater which is used in an aerosol inhaler is sufficiently provided, and local heat generation by the heater is reduced.

### Solution to Problem

In order to solve the above problems, a cartridge for aerosol inhaler according to the present invention includes the features of claim 1 and in particular:
a liquid reservoir that stores an aerosol-generating liquid, and
a heat-generating sheet that is provided with a positive electrode and a negative electrode, and that atomizes the aerosol-generating liquid supplied from the liquid reservoir, by generating heat when a current flow is caused between the positive electrode and the negative electrode, wherein
the heat-generating sheet is formed of a porous material, and a slit is provided so as to form a meandering electric path unit that is formed into a meandering shape while inhibiting localization in a current density of current flowing between the positive electrode and the negative electrode.

### Advantageous Effects of Invention

The present invention can provide a technology of a heat-generating sheet for aerosol inhaler for use in atomizing an aerosol-generating liquid such that a resistance value demanded of a heater which is used in the aerosol inhaler is sufficiently provided, and local heat generation by the heater is reduced.

### Brief Description of the Drawings

[Fig. 1] Fig. 1 is a schematic view of an electric cigarette as one example of an aerosol inhaler according to a first embodiment.
[Fig. 2] Fig. 2 is a schematic view of the electric cigarette as one example of the aerosol inhaler according to the first embodiment.
[Fig. 3] Fig. 3 is a view illustrating the schematic configuration of a cartridge according the first embodiment.
[Fig. 4] Fig. 4 is a view illustrating a plane structure of a porous heat-generating sheet according to the first embodiment.
[Fig. 5] Fig. 5 is a plan view of a heater unit of the porous heat-generating sheet according to the first embodiment.
[Fig. 6] Fig. 6 is a view illustrating, as a virtual line, an electric force line obtained when a current flow is caused to a heater unit of the porous heat-generating sheet according to the first embodiment.
[Fig. 7] Fig. 7 is a plan view of a heater unit of a porous heat-generating sheet according a first modification of the first embodiment.
[Fig. 8] Fig. 8 is a plan view of a heater unit of a porous heat-generating sheet according a second modification of the first embodiment.
[Fig. 9] Fig. 9 is a plan view of a heater unit of a porous heat-generating sheet according a third modification of the first embodiment.
[Fig. 10] Fig. 10 is a plan view of a heater unit of a porous heat-generating sheet according a fourth modification of the first embodiment.
[Fig. 11] Fig. 11 is a plan view of a heater unit of a porous heat-generating sheet according a fifth modification of the first embodiment.
[Fig. 12] Fig. 12 is a view illustrating a cartridge according to a second embodiment.
[Fig. 13] Fig. 13 is a view illustrating a cartridge according to a third embodiment.
[Fig. 14] Fig. 14 is a view illustrating a cartridge according to a fourth embodiment.
[Fig. 15] Fig. 15 is a view illustrating a cartridge according to a fifth embodiment.
[Fig. 16] Fig. 16 is a view illustrating a cartridge according to a first modification of the fifth embodiment.
[Fig. 17] Fig. 17 is a view illustrating a cartridge according to a second modification of the fifth embodiment.
[Fig. 18A] Fig. 18A is a view illustrating an electric cigarette according to a sixth embodiment.
[Fig. 18B] Fig. 18B is a view illustrating a cartridge according to the sixth embodiment.
[Fig. 19A] Fig. 19A is a view illustrating an electric cigarette according to a modification of the sixth embodiment.
[Fig. 19B] Fig. 19B is a view illustrating a cartridge according to the modification of the sixth embodiment.

### Description of Embodiments

Here, a description based on the drawings is given of embodiments of an aerosol inhaler and a cartridge and a porous heat-generating sheet applied to the aerosol inhaler according to the present invention. The dimensions, materials, shapes, and relative arrangement, etc. of components disclosed in the embodiments are not intended to limit the technical scope of the invention to these embodiments, unless otherwise particularly stated.

### <First embodiment>

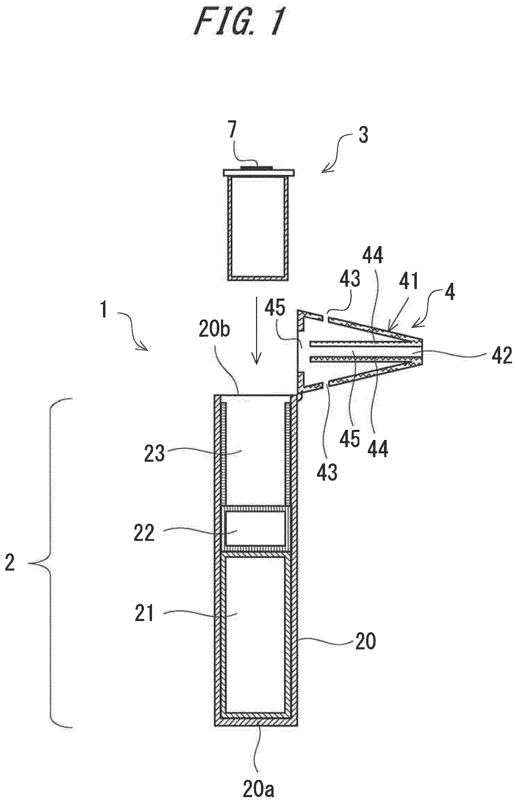

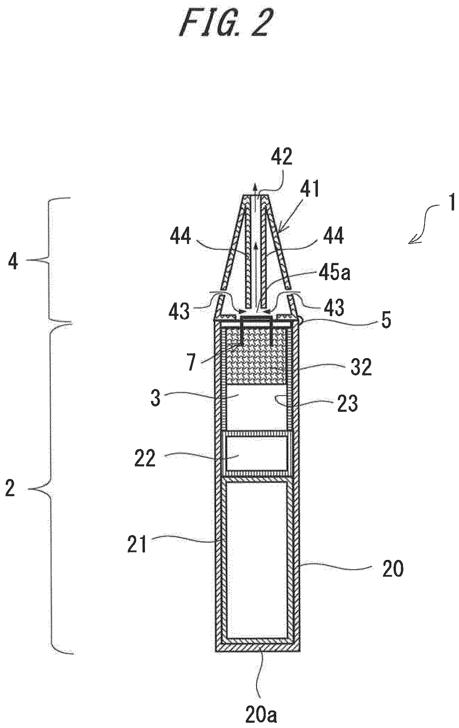

Figs. 1 and 2 are schematic views of an electric cigarette 1 as one example of an aerosol inhaler (flavor inhaler) according to a first embodiment. The electric cigarette 1 includes a body unit 2 and a mouthpiece unit 4. The body unit 2 has a body-side housing 20. A battery 21, an electronic control unit 22, and the like are accommodated in the body-side housing 20. The battery 21 may be a rechargeable battery such as a lithium ion secondary battery, for example.

The electronic control unit 22 is a computer that controls the entirety of the electric cigarette 1. The electronic control unit 22 may be a microcontroller having a circuit board (not illustrated) having a processor, a memory, and the like mounted thereon, for example.

The body-side housing 20 is a cylindrical bottomed shell, for example. From a bottom surface 20a side thereof, the battery 21 and the electronic control unit 22 are arranged in order. On an opening end 20b side located at the upper end of the body-side housing 20, a hollow accommodating cavity 23 for accommodating the cartridge 3 is formed. The cartridge 3 is an assembly formed by integrating a liquid tank (liquid reservoir) in which an aerosol-generating liquid for generating aerosols by being atomized through electric heating is accommodated and a porous heat-generating sheet that heats and atomizes the aerosol-generating liquid. The details thereof will be described later. In the electric cigarette 1 according to the present embodiment, the electronic control unit 22 and the battery 21 may be provided in this order from the bottom surface 20a side, and display means such as an LED or a display may be provided at an arbitrary position on the bottomed cylindrical shell.

The electronic control unit 22 and the battery 21 are connected to each other via an electric wire. The electronic control unit 22 controls power supply from the battery 21 to the porous heat-generating sheet as an atomizing unit of the cartridge 3. For example, the body-side housing 20 may be provided with a smoking switch (not illustrated) to be operated by a user. The smoking switch is connected to the electronic control unit 22 via an electric wire. When the electronic control unit 22 detects that the smoking switch has been operated to an ON state, the electronic control unit 22 controls the battery 21 so as to cause the battery 21 to supply power to the porous heat-generating sheet of the cartridge 3.

Next, the mouthpiece unit 4 is described. The mouthpiece unit 4 is hinge-connected to the body unit 2 with a hinge 5. Fig. 1 illustrates a state where the mouthpiece unit 4 is arranged at an open position in order to allow exchange (accommodation, removal) of the cartridge 3 with respect to the accommodating cavity 23 of the body unit 2. In the state where the mouthpiece unit 4 is arranged at the open position, the accommodating cavity 23 is in a state of being opened to the outside.

Meanwhile, Fig. 2 illustrates a state where the mouthpiece unit 4 is arranged at a closed position at which the mouthpiece unit 4 is rotated from the open position by approximately 90 degrees. In the state where the mouthpiece unit 4 is arranged at the closed position, the mouthpiece unit 4 is in a state of covering the accommodating cavity 23 and the upper portion of the cartridge 3 accommodated in the accommodating cavity 23. However, in the electric cigarette 1 according to the present embodiment, the mouthpiece unit 4 and the body unit 2 (battery assembly) may be attachable to and detachable from each other. In this case, means for joining the mouthpiece unit 4 and the body unit 2 together is not limited to particular means. Known connecting means including connection using a screw, connection through a sleeve member, or fitting connection, for example, can be used.

The mouthpiece unit 4 has a housing 41. The housing 41 of the mouthpiece unit 4 has a shape tapered toward a tip end so as to be easily put into a user's mouth. At the tip end side thereof, an inhalation port 42 is formed. Further, an air intake port 43 is provided to the housing 41 of the mouthpiece unit 4. Moreover, a cylindrical baffle wall 44 connected to the inhalation port 42 is provided in the housing 41 of the mouthpiece unit 4. An inner passage 45 is formed by the baffle wall 44. The inner passage 45 in the mouthpiece unit 4 communicates with the inhalation port 42 and the air intake port 43. When a user smokes the cigarette, outside air taken into the housing 41 from the outside through the air intake port 43 flows through the inner passage 45 and reaches the inhalation port 42. In the inner passage 45, an atomizing cavity 45a is formed near the upper surface of the cartridge 3. The cartridge 3 vaporizes the aerosol-generating liquid stored in the liquid tank through electric heating, mixes the vaporized aerosol-generating liquid with air in the atomizing cavity 45a, and thereby generates aerosols. The generated aerosols are guided to the inhalation port 42 via the atomizing cavity 45a and the inner passage 45. The user can inhale the aerosols through the inhalation port 42.

In the electric cigarette 1, instead of the smoking switch, an inhalation sensor (not illustrated) may be provided to the body-side housing 20, and user's inhaling (puffing) at the inhalation port 42 may be detected by the inhalation sensor, whereby the user's smoking request may be detected.

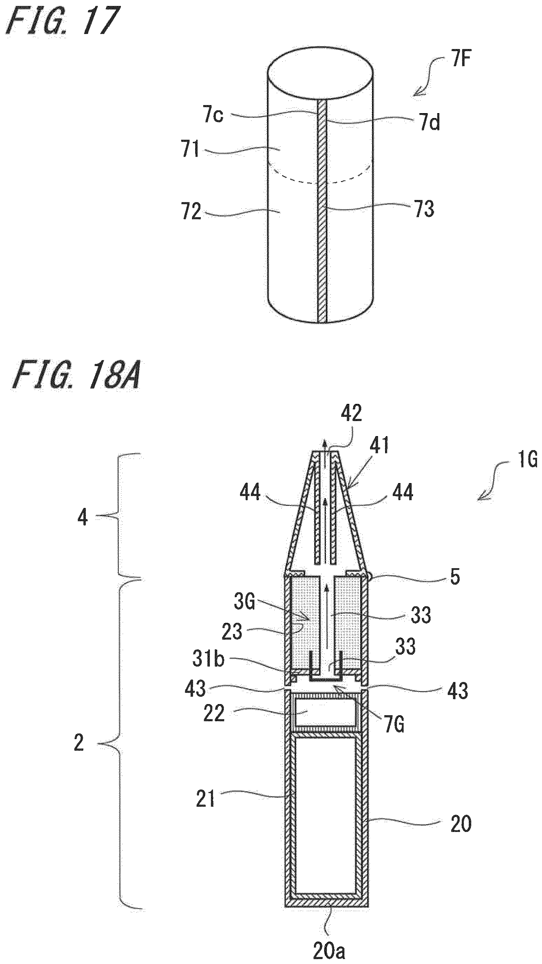

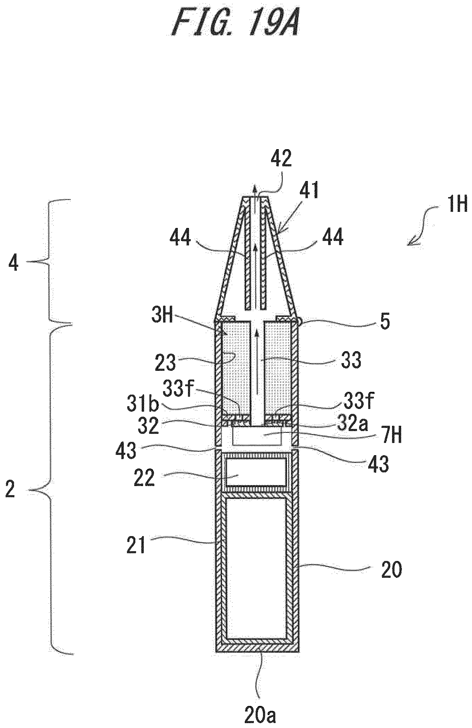

In this case, the inhalation sensor may be connected to the electronic control unit 22 via an electric wire. When the user's inhaling (puffing) at the inhalation port 42 is detected by the inhalation sensor, the electronic control unit 22 may control the battery 21 so as to cause the battery 21 to supply power to the porous heat-generating sheet (described later) of the cartridge 3. The present invention may use, as the inhalation sensor, a pressure sensitive sensor that detects negative pressure caused by user's inhaling, or a thermal flow meter (MEMS flow sensor, etc.). The atomizing cavity 45a is provided in the mouthpiece unit 4, but the accommodating cavity 23 on the body unit 2 (battery assembly) side may be made deeper such that the atomizing cavity 45a is provided to the body unit 2. In this case, the air intake port 43 is preferably provided to the body unit 2 (see Figs. 18A, 19A, etc.) too.

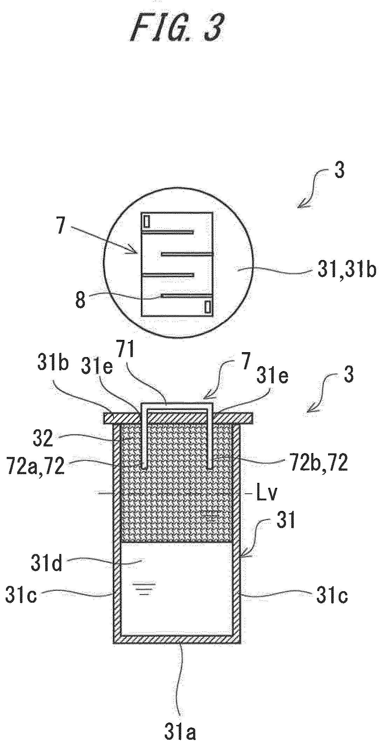

Fig. 3 is a view illustrating the schematic configuration of the cartridge 3 according to the present embodiment. In Fig. 3, the upper stage illustrates the upper surface of the cartridge 3, and the lower stage illustrates a longitudinal cross section of the cartridge 3. In the present embodiment where the cartridge 3 has the liquid tank 31 accommodating the aerosol-generating liquid therein, the liquid tank 31 is a cylindrical bottle case having a circular bottom 31a, a circular lid 31b, and a tubular lateral wall 31c. However, the shape of the liquid tank 31 is not limited to a particular shape. A liquid reserving space 31d that stores the aerosol-generating liquid is formed in the liquid tank 31. The aerosol-generating liquid is stored in the liquid reserving space 31d. The aerosol-generating liquid may be a mixture liquid of glycerol (G), propylene glycol (PG), a nicotine liquid, water, a flavoring agent, or the like, for example. The mixture ratio of the materials contained in the aerosol-generating liquid may be changed, as appropriate. In the present invention, the aerosol-generating liquid does not need to contain a nicotine liquid.

A liquid supply member 32 that supplies the aerosol-generating liquid to the porous heat-generating sheet (described later) is provided on the upper side of the liquid reserving space 31d in the liquid tank 31. The liquid supply member 32 may be cotton fibers, for example. In the present embodiment, the liquid supply member 32 may be fixed to the rear surface of the lid 31b of the liquid tank 31, for example. The present invention may not be provided with the liquid supply member 32. Reference character 7 in Fig. 3 denotes the porous heat-generating sheet that heats and atomizes the aerosol-generating liquid stored in the liquid tank 31. Reference character Lv in Fig. 3 denotes the initial liquid level of the aerosol-generating liquid stored in the liquid tank 31 (liquid reserving space 31d). A predetermined amount of the aerosol-generating liquid is stored in the liquid tank 31 (liquid reserving space 31d) in producing of the electric cigarette 1 so that the liquid level of the aerosol-generating liquid is adjusted to the initial liquid level Lv. When the initial liquid level Lv is set above the liquid supply member 32, in other words, when the aerosol-generating liquid is put to reach a level above the lower end of the liquid supply member 32, the aerosol-generating liquid can be stably supplied to the porous heat-generating sheet.

The porous heat-generating sheet 7 is bent into a substantially C-like shape in a side view. When not in use, at least a portion of the porous heat-generating sheet 7 is in direct contact or indirect contact via the liquid supply member 32 with the aerosol-generating liquid in the liquid tank 31 (liquid reserving space 31d). The porous heat-generating sheet 7 is a wick heater having both a function as a wick of directly or indirectly suctioning the aerosol-generating liquid stored in the liquid tank 31 and holding the aerosol-generating liquid and a function as a heater of atomizing the held aerosol-generating liquid through electric heating when a user smokes the cigarette. The porous heat-generating sheet 7 includes a flat plate-like heater unit 71 that is arranged so as to face the front surface of the lid 31b of the liquid tank 31, and a first suctioning unit 72a and a second suctioning unit 72b that are downwardly bent from the heater unit 71. Hereinafter, the first suctioning unit 72a and the second suctioning unit 72b are collectively referred to as "suctioning units 72".

Insertion holes 31e through which the suctioning units 72 are inserted into the liquid tank 31 are formed in the lid 31b of the liquid tank 31. The suctioning units 72 are inserted in the liquid reserving space 31d side through the insertion holes 31e. In the present embodiment, both sides of the heater unit 71 are bent such that the pair of suctioning units 72 are continuous to the heater unit 71. However, the number of the suctioning units 72 is not limited to a particular number. Each end of the suctioning units 72 may extend to the interior of the liquid supply member 32 made of cotton fibers, for example, as illustrated in Fig. 3, or may extend toward the liquid reserving space 31d side in a state of penetrating the liquid supply member 32. In the present invention, the components may be arranged such that a portion of each of the suctioning units 72 abuts on a surface of the liquid supply member. The contact area between the suctioning units 72 and the liquid supply member 32, or the contact surface (e.g., the upper end surface or a side circumference surface of the liquid supply member 32) of the suctioning units 72 with respect to the liquid supply member 32 can be changed, as appropriate.

The porous heat-generating sheet 7 can at least temporarily hold the aerosol-generating liquid. A material that is used for the porous heat-generating sheet 7 is not limited to a particular material as long as the porous heat-generating sheet 7 can be used as a wick heater that atomizes the held aerosol-generating liquid through electric heating when a user smokes the cigarette. The porous heat-generating sheet 7 may be a porous metal body containing nickel, nichrome, stainless steel (SUS), or the like, for example. Further, as long as a conductive material capable of generating heat when power is applied thereto may be used, ceramic such as silicon carbide (SiC) may be used therefor. The porous heat-generating sheet 7 of the present embodiment has a three-dimensional network structure. The three-dimensional network structure has a structure including voids at least some of which are connected to each other, that is, an open-cell structure. The porous heat-generating sheet 7 of the present embodiment thus configured, has a function of suctioning a liquid by a capillary phenomenon. Examples of the porous metal body having such an open-cell structure include celmet (product name) manufactured by Sumitomo Electric Industries, Ltd. Celmet (product name) is a porous metal body containing nickel (Ni) or a porous metal body containing an alloy of nickel and chromium (Cr).

The thickness of the porous heat-generating sheet 7 of the present embodiment is preferably 0.1 to 3.0 mm, and is more preferably 0.2 to 1.0 mm. The total area of a portion, of the porous heat-generating sheet 7, which functions as a heater is preferably 1 to 250 mm², and is more preferably 3 to 150 mm². In a case where the porous heat-generating sheet 7 has a rectangular shape, the aspect ratio (longer side: shorter side) of the portion that functions as a heater is preferably 1:1 to 3:1, and is more preferably 1:1 to 2:1. The number of linear electric path portions included in the porous heat-generating sheet 7 is preferably 2 to 20, and is more preferably 5 to 15. The number of bent electric path portions of a meandering electric path included in the porous heat-generating sheet 7 is preferably 1 to 19, and is more preferably 4 to 14.

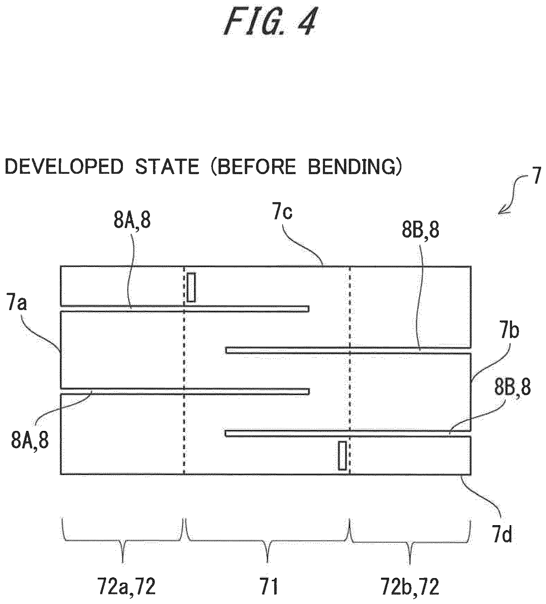

Fig. 4 is a view illustrating a plan structure of the porous heat-generating sheet 7 of the present embodiment. Fig. 4 illustrates a state where the porous heat-generating sheet 7 is developed, that is, a state before the suctioning units 72 are bent with respect to the heater unit 71. Broken lines in Fig. 4 indicate the boundaries between the heater unit 71 and the suctioning units 72.

In the example illustrated in Fig. 4, the porous heat-generating sheet 7 has a flat rectangular shape. The shape of the porous heat-generating sheet 7 is not limited to a particular shape. The porous heat-generating sheet 7 may have a parallelogram shape, a diamond shape, or the like. Reference characters 7a, 7b, 7c, 7d denote the left side, the right side, the upper side, and the lower side of the porous heat-generating sheet 7. A plurality of slits 8 extending in parallel to the upper side 7c and the lower side 7d are provided in the porous heat-generating sheet 7. Hereinafter, the direction, in the porous heat-generating sheet 7, along the upper side 7c and the lower side 7d is referred to as a lateral width direction of the porous heat-generating sheet 7. Also, the direction, in the porous heat-generating sheet 7, along the left side 7a and the right side 7b is referred to as the up/down direction of the porous heat-generating sheet 7.

The slits 8 are cutouts penetrating the porous heat-generating sheet 7 in the thickness direction. The slits 8 may be produced by a laser cutting method, for example, but the production method therefor is not limited to a particular method. The slits 8 may be produced by punching. A laser cutting method is effective particularly for producing narrow slits. The slits 8 may be formed in the porous heat-generating sheet 7 by a YAG laser, a CO₂ laser, or the like, for example. The width dimension of each of the slits 8 is not limited to a particular dimension. The width dimension of each of the slits 8 is a dimension in a direction orthogonal to the length dimension of the slit 8 extending in the lateral width direction of the porous heat-generating sheet 7.

In the example illustrated in Fig. 4, the slits 8 extend in parallel from the left side 7a and the right side 7b of the porous heat-generating sheet 7 toward the center side in the lateral width direction of the heater unit 71. Hereinafter, the slits 8 extending from the left side 7a of the porous heat-generating sheet 7 are also referred to as "first slits 8A", and the slits 8 extending from the right side 7b of the porous heat-generating sheet 7 are also referred to as "second slits 8B". As illustrated in Fig. 4, the first slits 8A and the second slits 8B are alternately provided in the porous heat-generating sheet 7. The ends of the first slits 8A extend to reach a right side 7b side region across the center portion, in the lateral width direction, of the heater unit 71. On the other hand, the ends of the second slits 8B extend to reach a left side 7a side region across the center portion, in the lateral width direction, of the heater unit 71. As a result of this, a state where the ends of the first slits 8A and the second slits 8B overlap each other in the slit extension direction is obtained.

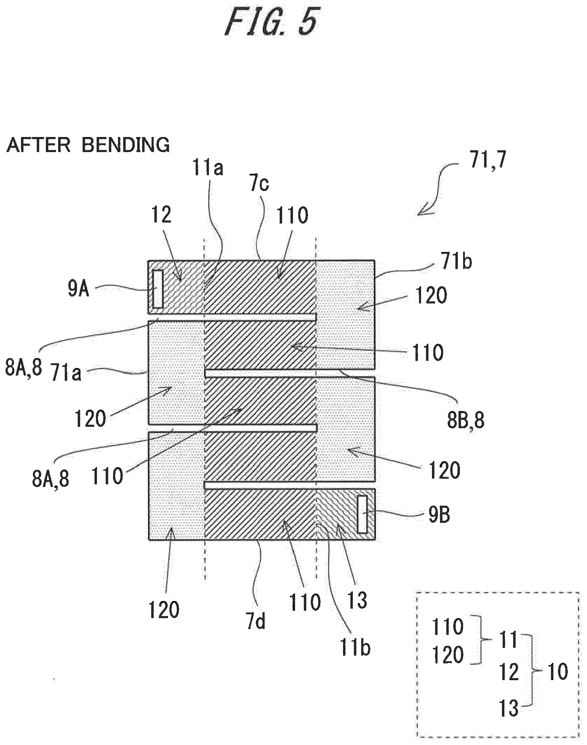

Fig. 5 is a plan view of the heater unit 71 of the porous heat-generating sheet 7 according to the first embodiment. Reference character 71a in Fig. 5 denotes a first end edge located at the bending boundary between the heater unit 71 and the first suctioning unit 72a. Reference character 71b in Fig. 5 denotes a second end edge located at the bending boundary between the heater unit 71 and the second suctioning unit 72b. As illustrated in Fig. 5, a positive electrode 9A and a negative electrode 9B are provided to the heater unit 71 of the porous heat-generating sheet 7. The positive electrode 9A and the negative electrode 9B on the heater unit 71 are connected to the battery 21 arranged in the body unit 2 via a lead wire, etc. When power is supplied from the battery 21 to the porous heat-generating sheet 7 on the basis of a control signal from the electronic control unit 22, a current flow is caused to an electric path 10 connecting the positive electrode 9A and the negative electrode 9B on the heater unit 71. Thus, the heater unit 71 generates heat.

As illustrated in Fig. 5, the electric path 10 connecting the positive electrode 9A and the negative electrode 9B on the heater unit 71 is formed into a meandering shape by the slits 8. More specifically, the electric path 10 includes a meandering electric path unit 11 that is formed into a meandering shape by sequentially connecting linear electric path portions 110 each having a linear shape and bent electric path portions 120 formed by bending the linear electric path portions 110, includes a positive electrode-provided electric path unit 12 connected (continuous) to one end 11a of the meandering electric path unit 11, and includes a negative electrode-provided electric path unit 13 connected (continuous) to the other end 11b of the meandering electric path unit 11. Here, the positive electrode 9A is provided on the positive electrode-provided electric path unit 12, and the negative electrode 9B is provided on the negative electrode-provided electric path unit 13. The positive electrode-provided electric path unit 12 may be substantially equivalent to a region occupied by the positive electrode 9A, but the positive electrode 9A may be arranged in a portion of the positive electrode-provided electric path unit 12. Also, the negative electrode-provided electric path unit 13 may be substantially equivalent to a region occupied by the negative electrode 9B, but the negative electrode 9B may be provided in a portion of the negative electrode-provided electric path unit 13.

The meandering electric path unit 11 is formed into a meandering shape by sequentially and alternately connecting the linear electric path portions 110 and the bent electric path portions 120. The number of the linear electric path portions 110 and the bent electric path portions 120 constituting the meandering electric path unit 11 is not limited to a particular number. However, from the viewpoint of ensuring of the electric path length of the meandering electric path unit 11 and improvement of the electric resistance, the number of the linear electric path portions 110 and the bent electric path portions 120 included in the meandering electric path unit 11 is preferably greater.

In Fig. 5, the linear electric path portions 110 of the meandering electric path unit 11 are hatched with oblique lines, and the bent electric path portions 120 are hatched with dots. The positive electrode-provided electric path unit 12 and the negative electrode-provided electric path unit 13 are hatched with wavy lines. In Fig. 5, the meandering electric path unit 11 is formed of five linear electric path portions 110 hatched with oblique lines and four bent electric path portions 120 hatched with dots. In the example illustrated in Fig. 5, the meandering electric path unit 11 has a plurality of the linear electric path portions 110, and the linear electric path portions 110 are separated by the slits 8 (first slits 8A, second slits 8B). As is clear from Fig. 5, in the heater unit 71 of the present embodiment, the slits 8 (first slits 8A, second slits 8B) extend in the extension direction of the linear electric path portions 110 of the meandering electric path unit 11.

In the example illustrated in Fig. 5, the one end 11a of the meandering electric path unit 11 is formed by a linear electric path portion 110, and the positive electrode-provided electric path unit 12 is connected to the linear electric path portion 110 that is located on the one end 11a side. However, the one end 11a side of the meandering electric path unit 11 may be formed by a bent electric path portion 120, and the positive electrode-provided electric path unit 12 may be connected to the bent electric path portion 120 located on the one end 11a side. In the example illustrated in Fig. 5, the other end 11b of the meandering electric path unit 11 is formed by a linear electric path portion 110, and the negative electrode-provided electric path unit 13 is connected to the linear electric path portion 110 that is located on the other end 11b side. However, the other end 11b side of the meandering electric path unit 11 may be formed by a bent electric path portion 120, and the negative electrode-provided electric path unit 13 may be connected to the bent electric path portion 120 that is located on the other end 11b side.

Since the porous heat-generating sheet 7 thus configured has a function of suctioning a liquid by a capillary phenomenon, the suctioning unit 72 inserted in the liquid reserving space 31d in the liquid tank 31 suctions the aerosol-generating liquid stored in the liquid reserving space 31d directly from the liquid reserving space 31d or indirectly through the liquid supply member 32 (see Fig. 3). The aerosol-generating liquid having been suctioned from the liquid reserving space 31d by the suctioning units 72 is also transmitted from the suctioning units 72 to the heater unit 71, and is held by the porous heat-generating sheet 7.

Here, the user performs an operation of pressing a smoking switch (not illustrated) when the user smokes the cigarette. When the electronic control unit 22 detects the ON state of the smoking switch, the electronic control unit 22 outputs a control signal to the battery 21 so as to cause the battery 21 to supply power to the porous heat-generating sheet 7 of the cartridge 3. As a result, current flows through the electric path 10 connecting the positive electrode 9A and the negative electrode 9B on the heater unit 71 of the porous heat-generating sheet 7 so that a current flow is caused and heat is generated. In this regard, according to the heater unit 71 of the present embodiment, the meandering electric path unit 11 is formed by the slits 8 provided in the flat plate-like heater unit 71, and thus, the electric path length of the electric path 10 connecting the positive electrode 9A and the negative electrode 9B can be favorably increased. Thus, the electric resistance of a portion between the positive electrode 9A and the negative electrode 9B can be increased. In addition, according to the porous heat-generating sheet 7 of the present embodiment, the electric resistance can be further increased per unit area of a porous heat-generating sheet that functions as an electric path, compared to a case where no slits are provided. As a result of this, when a current flow to the heater unit 71 is caused, the heat generation amount by the heater unit 71 can be sufficiently ensured. Accordingly, the aerosol-generating liquid can be sufficiently heated and can be smoothly atomized by the heater unit 71.

In particular, according to the heater unit 71 of the porous heat-generating sheet 7 of the present embodiment, the meandering electric path unit 11 has a plurality of the linear electric path portions 110, the linear electric path portions 110 are separated from each other by the slits 8 (first slits 8A, second slit 8B), and the slits 8 are provided so as to extend in the extension direction of the linear electric path portions 110 of the meandering electric path unit 11. Accordingly, the electric path length can be more effectively ensured, and thus, the effect of increasing the electric resistance of the portion between the positive electrode 9A and the negative electrode 9B on the heater unit 71 can be more easily obtained.

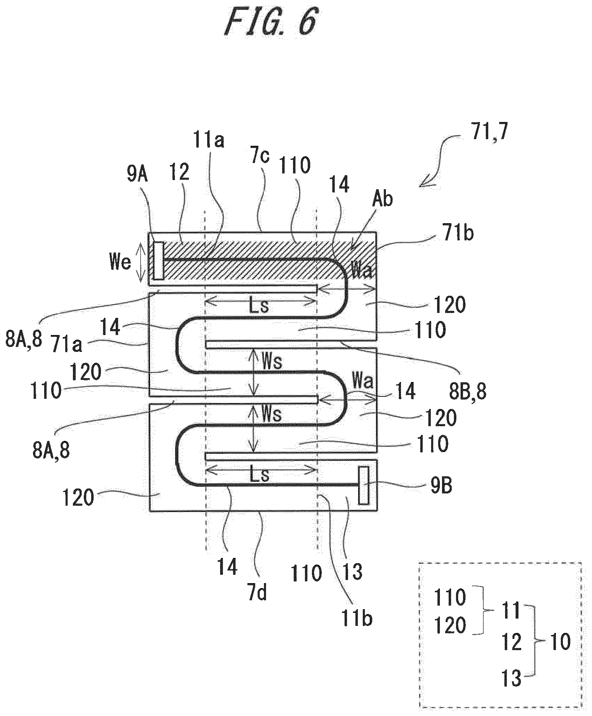

Here, Fig. 6 is a view illustrating, as a virtual line, an electric force line 14 obtained when a current flow to the heater unit 71 of the porous heat-generating sheet 7 is caused. As illustrated in Fig. 6, according to the heater unit 71 of the present embodiment, the bent electric path portions 120 are not continuous to one another but the linear electric path portions 110 are connected between the bent electric path portions 120 (in other words, the bent electric path portions 120 and the linear electric path portions 110 are sequentially and alternately connected). Therefore, an abrupt change of the direction of the electric force line 14 can be reduced while the electric path length is increased. Accordingly, while the electric resistance is increased per unit volume of the heater unit 71, the distribution of the electric field intensity can be made less likely to become nonuniform. As a result of this, while the heat generation amount by the heater unit 71 of the porous heat-generating sheet 7 when a current flow is being caused, is sufficiently ensured, local heat generation by the heater unit 71 can be made less likely to occur. That is, in the porous heat-generating sheet 7 of the present embodiment, while localization in the current density of current flowing between the positive electrode 9A and the negative electrode 9B is inhibited, the slits 8 are provided such that the meandering electric path unit 11 that is formed into a meandering shape is formed. Accordingly, the heater unit 71 can have a sufficient resistance value, and local heat generation by the heater unit 71 can be reduced.

Reference character Ls in Fig. 6 denotes the length (hereinafter, referred to as "slit overlapping length") of an overlapping section where the adjacent first slit 8A and second slit 8B among the slits 8 overlap each other in the extension direction thereof. Reference character Ws denotes an internal dimension (hereinafter, referred to as "slit interval") by which the adjacent first slit 8A and second slit 8B among the slits 8 are separated. The slit interval Ws corresponds to the electric path width of a linear electric path portion 110 sandwiched between the adjacent first slit 8A and second slit 8B. Reference character Wa denotes the electric path width of a bent electric path portion 120 of the meandering electric path unit 11. Reference character We denotes an electrode effective width of the positive electrode 9A. The electrode effective width We of the positive electrode 9A is the width dimension of the positive electrode 9A in a direction orthogonal to a direction in which current flows out from the positive electrode 9A to the electric path 10 (positive electrode-provided electric path unit 12). In the porous heat-generating sheet 7 of the present embodiment, the aforementioned electrode effective width We is designed to a dimension equal to or smaller than the electric path width of a minimum electric path width portion where the electric path width of the electric path 10 connecting the positive electrode 9A and the negative electrode 9B becomes narrowest, as illustrated in Fig. 6. The electric path width of the electric path 10 is a dimension in a direction substantially orthogonal to the direction in which current flows through the electric path 10. In the example illustrated in Fig. 6, the dimension of the slit interval Ws is set to be equal to the dimension of the electric path width Wa of the bent electric path portion 120. The dimension corresponds to the electric path width of the minimum electric path width portion where the electric path width of the electric path 10 becomes narrowest. That is, in the present embodiment, the electric path width Wa and the slit interval Ws of the bent electric path portions 120 are each set to a dimension relatively larger than the electrode effective width We of the positive electrode 9A.

If the width dimension of the electric path 10 is not sufficiently ensured with respect to the electrode effective width We, that is, if the electric path partially includes an area having a width dimension smaller than the electrode effective width We, localization in the current density is likely to occur at the area having such a small width dimension. In contrast, in the present embodiment, the electric path width (the electric path width Wa, the slit interval Ws of the bent electric path portions 120) of the minimum electric path width portion of the electric path 10 is set to a dimension relatively larger than the electrode effective width We of the positive electrode 9A. Consequently, in the porous heat-generating sheet 7, localization in the current density of current flowing between the electrodes can be inhibited, and local heat generation by the heater unit 71 can be more effectively reduced.

Furthermore, as illustrated in Fig. 6, the porous heat-generating sheet 7 of the present embodiment has a structure in which the positive electrode 9A is formed, within the plane of the porous heat-generating sheet 7, so as to extend in a direction (i.e., a direction in which current flows out from the positive electrode 9A to the electric path 10 (positive electrode-provided electric path unit 12)) orthogonal to the direction of the electrode effective width We, and a band-like virtual band region Ab (the hatched region in Fig. 6) having a width equal to the electrode effective width We does not include the ends of the slits 8 that extend from edges of the porous heat-generating sheet 7 to the inner side on the plane of the porous heat-generating sheet 7. If the virtual band region includes any end of the slits, a region where a current flow is inhibited/deformed by the slits and a region on which no influence is exerted by the slits are generated. That is, the disorder in the electric force line results in nonuniform heat generation. In contrast, the present embodiment adopts the structure in which the ends of the slits 8 extending from edges of the porous heat-generating sheet 7 to the inner side are not included in the virtual band region Ab. Consequently, disorder in the electric force line in the heater unit 71 can be inhibited, and uniform heat generation by the heater unit 71 can be facilitated.

Here, in the heater unit 71 of the porous heat-generating sheet 7, the slit overlapping length Ls is set to be equal to or longer than the slit interval Ws. The slit overlapping length Ls is substantially equal to the length of a linear electric path portion 110. Therefore, when the slit overlapping length Ls is ensured to be at least equal to or longer than the slit interval Ws, the electric path length of the meandering electric path unit 11 can be easily ensured. Furthermore, in the heater unit 71, a plurality of the linear slits 8 separating the linear electric path portions 110 from one another are arranged in parallel at a fixed interval. That is, the slits 8 are arranged in parallel, and the slit interval Ws therebetween is fixed. Accordingly, the electric path width of the meandering electric path unit 11 in the heater unit 71 can be made substantially fixed (see Figs. 5 and 6, etc.). As a result of this, local heat generation by the heater unit 71 is less likely to occur in the meandering electric path unit 11. Thus, uniform heat generation by the entirety of the heater unit 71 can be facilitated.

In the heater unit 71 according to the present embodiment, the electric path length of the meandering electric path unit 11 is set to be equal to or longer than the dimension of a straight line connecting the positive electrode 9A and the negative electrode 9B on the heater unit 71. With this configuration, an effect of increasing the electric resistance per unit volume of the heater unit 71 can be more easily obtained. In the present invention, the total value (∑Ls) of the slit overlapping lengths Ls is set to be equal to or greater than the dimension of the straight line connecting the positive electrode 9A and the negative electrode 9B on the heater unit 71. According to the porous heat-generating sheet 7 of the present embodiment, the balance between the liquid amount of an aerosol-generating liquid which can be held in the porous heat-generating sheet 7 and a heat generation amount of heat which is generated by the porous heat-generating sheet 7 with standard power applied, can be kept.

### <Modification>

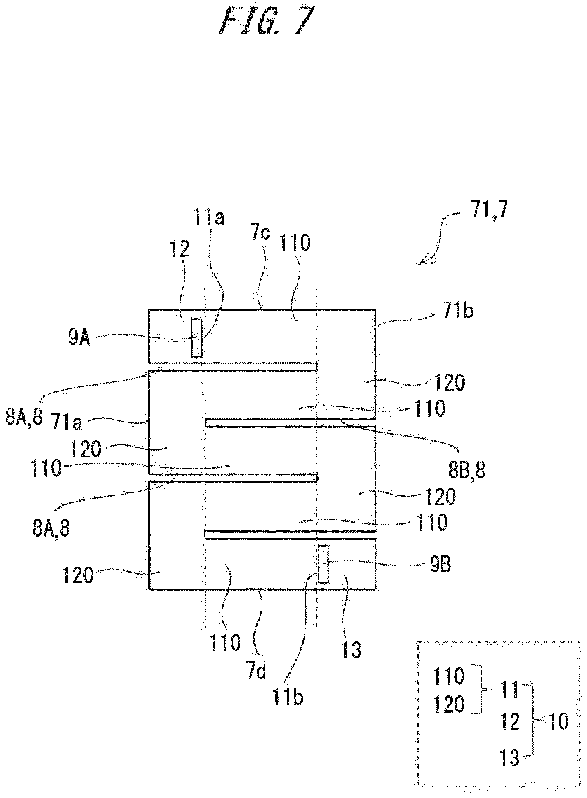

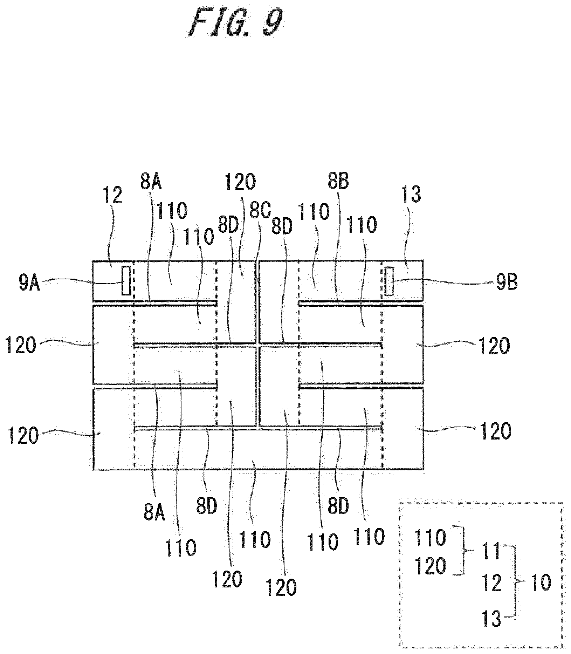

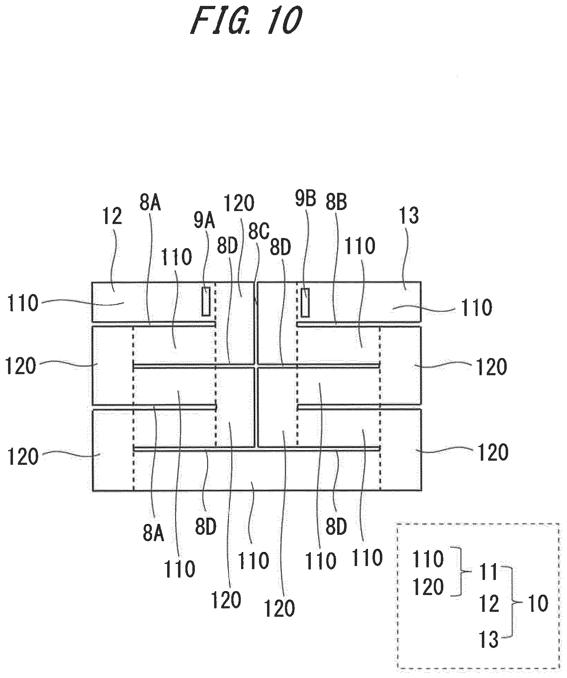

Next, a description is given of a modification of the porous heat-generating sheet 7 according to the present embodiment. Hereinafter, the components identical to those of the aforementioned embodiment are denoted by the same reference numerals, and a detailed description thereof is omitted. Figs. 7 to 11 are plan views of the heater unit 71 in the porous heat-generating sheet 7 according to first to fifth modifications.

In the first modification illustrated in Fig. 7, the positions of the positive electrode 9A and the negative electrode 9B arranged on the positive electrode-provided electric path unit 12 and the negative electrode-provided electric path unit 13 are different from those in the configuration example illustrated in Fig. 5. That is, in the first modification illustrated in Fig. 7, the positive electrode 9A on the positive electrode-provided electric path unit 12 is arranged at a position near the one end 11a of the meandering electric path unit 11. The negative electrode 9B on the negative electrode-provided electric path unit 13 is arranged at a position near the other end 11b of the meandering electric path unit 11. However, as in the configuration example illustrated in Fig. 5, the positive electrode 9A is preferably arranged at an end, of the positive electrode-provided electric path unit 12, opposite to the end connected to the one end 11a of the meandering electric path unit 11. With this configuration, the length of the electric path 10 in the heater unit 71 can be further increased. Similarly, the negative electrode 9B is preferably arranged at an end, of the negative electrode-provided electric path unit 13, opposite to the end connected to the other end 11b of the meandering electric path unit 11. With this configuration, the length of the electric path 10 in the heater unit 71 can be further increased.

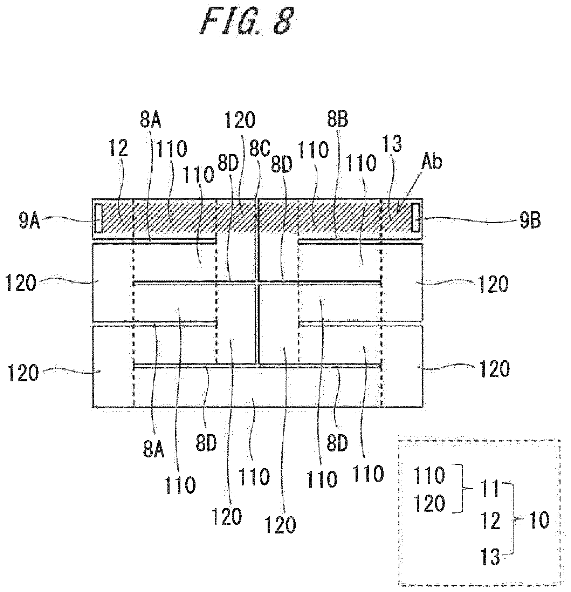

Further, as in the second to fourth modifications illustrated in Figs. 8 to 10, the meandering electric path unit 11 of the heater unit 71 may be formed by the slits 8 including, in addition to the aforementioned first slits 8A and second slits 8B, a longitudinal slit 8C extending in the up/down direction of the porous heat-generating sheet 7 (heater unit 71), a lateral slit 8D extending in the lateral width direction of the heater unit 71 from the longitudinal slit 8C, and the like. In this way, various modification patterns can be adopted for the electric path 10 in the heater unit 71 of the porous heat-generating sheet 7.

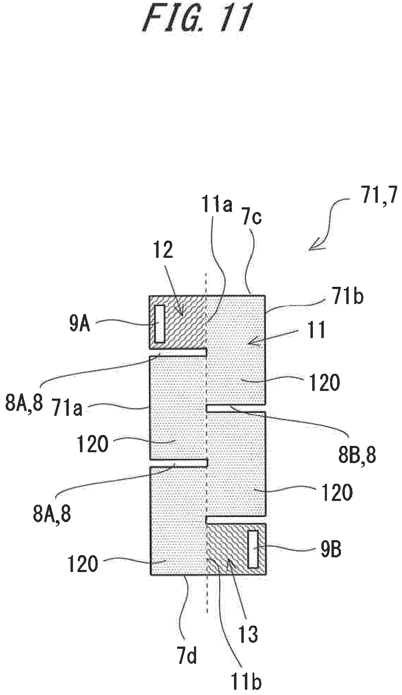

Various modifications can be made to the forms of the positive electrode 9A and the negative electrode 9B arranged on the positive electrode-provided electric path unit 12 and the negative electrode-provided electric path unit 13. For example, the shapes or sizes of the positive electrode 9A and the negative electrode 9B can be changed, as appropriate. The positive electrode 9A may be provided on the front surface or rear surface of the heater unit 71 of the porous heat-generating sheet 7. Similarly, the negative electrode 9B may be provided in the front surface or rear surface of the heater unit 71 of the porous heat-generating sheet 7. In Fig. 8, the virtual band region Ab is indicated as a hatched region. The porous heat-generating sheet 7 illustrated in Fig. 8 also has the structure in which the virtual band region Ab does not include the end of the slit 8 (longitudinal slit 8C in the example in Fig. 8) that extends from an edge of the porous heat-generating sheet 7 toward the inner side on the plane of the porous heat-generating sheet 7. In the heater unit 71 of the porous heat-generating sheet 7, the meandering electric path unit 11 may be formed by providing therein the slits 8 such that localization in the current density of current flowing between the positive electrode 9A and the negative electrode 9B is inhibited. Therefore, the meandering electric path unit 11 does not need to include any of the linear electric path portions 110. For example, as in the fifth modification illustrated in Fig. 11, the meandering electric path unit 11 may be formed by continuously connecting the bent electric path portions 120.

In the porous heat-generating sheet 7 according to the present embodiment described with reference to Figs. 3 to 5, etc., both sides of the heater unit 71 are bent so that the suctioning units 72 are continuous to the heater unit 71. However, the porous heat-generating sheet 7 is not limited to this. For example, the porous heat-generating sheet 7 may not include the suctioning units 72. Another alternative means may be used to suction the aerosol-generating liquid stored in the liquid tank 31 and supply the aerosol-generating liquid to the heater unit 71. For example, the porous heat-generating sheet 7 (heater unit 71) and the liquid supply member 32 in the liquid tank 31 may supply the aerosol-generating liquid in the liquid tank 31 to the porous heat-generating sheet 7 (heater unit 71) .

### <Second embodiment>

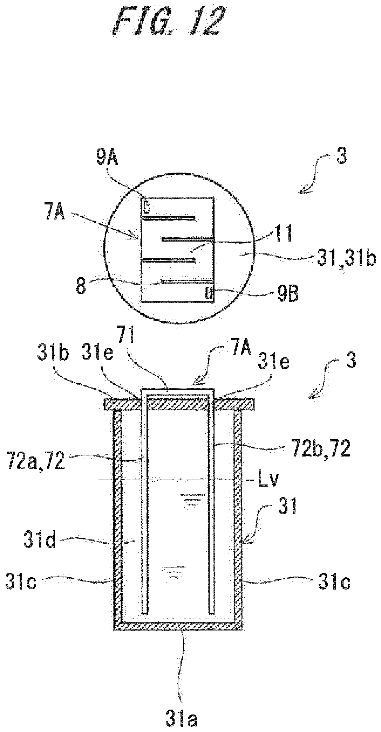

Fig. 12 is a view illustrating a cartridge 3A according to a second embodiment. In the cartridge 3A illustrated in Fig. 12, the liquid supply member 32 is not provided to the liquid tank 31 (liquid reserving space 31d). The porous heat-generating sheet 7A according to the second embodiment is configured such that the suctioning units 72 extend to an area near the bottom of the liquid tank 31, and the suctioning units 72 directly suction the aerosol-generating liquid stored in the liquid reserving space 31d.

### <Third embodiment>

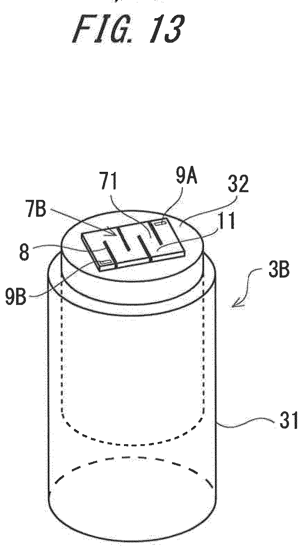

Fig. 13 is a view illustrating a cartridge 3B according to the third embodiment. A porous heat-generating sheet 7B in the cartridge 3A illustrated in Fig. 13 is formed of the heater unit 71 alone, and does not have any suctioning unit 72. In the cartridge 3B, the liquid supply member 32 formed into a cylindrical shape, for example, is provided to the liquid tank 31, and the porous heat-generating sheet 7B is placed on the upper surface of the liquid supply member 32. The heater unit 71 of the porous heat-generating sheet 7B has a structure identical to that of the heater unit 71 of the porous heat-generating sheet 7 of the first embodiment. The porous heat-generating sheet 7B of the present embodiment can suction the aerosol-generating liquid from the rear surface of the heater unit 71 which abuts on the upper surface of the liquid supply member 32, and can hold the aerosol-generating liquid. The shape of the liquid supply member 32 is not limited to the above example.

### <Fourth embodiment>

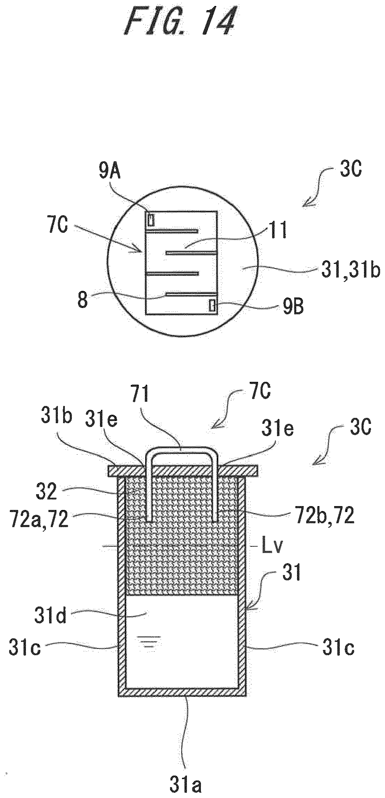

Fig. 14 is a view illustrating a cartridge 3C according to the fourth embodiment. A porous heat-generating sheet 7C according to the cartridge 3C is different from the porous heat-generating sheet 7 according to the first embodiment in that the porous heat-generating sheet 7C has a U-like shape in a side view while the porous heat-generating sheet 7 is bent into a substantially C-like shape in a side view. However, the other components in the porous heat-generating sheet 7C are identical to those of the porous heat-generating sheet 7.

### <Fifth embodiment>

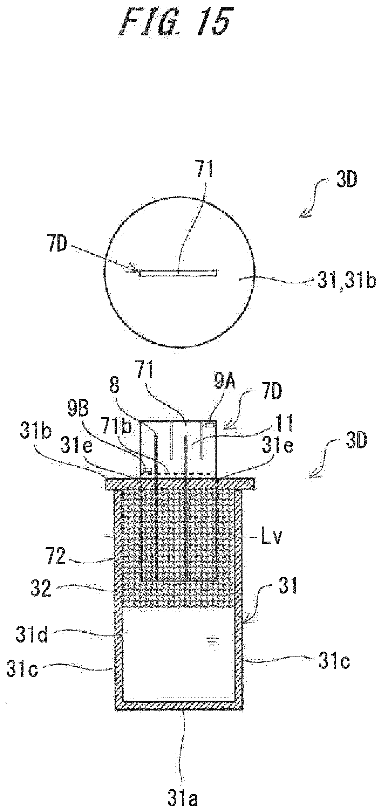

Fig. 15 is a view illustrating a cartridge 3D according to the fifth embodiment. In a porous heat-generating sheet 7D according to the cartridge 3D, one suctioning unit 72 is connected to the right side 71b of the heater unit 71. The other components of the porous heat-generating sheet 7D are identical to those of the porous heat-generating sheet 7 according to the first embodiment.

The porous heat-generating sheet 7D entirely has a flat plate-like shape. The suctioning unit 72 is inserted in the liquid reserving space 31d through the insertion hole 31e formed in the lid 31b of the liquid tank 31. That is, in the cartridge 3D, the porous heat-generating sheet 7D is set with respect to the liquid tank 31 while the heater unit 71 of the flat plate-like porous heat-generating sheet 7D is exposed to the outside of the liquid tank 31 and the suctioning unit 72 is inserted outside the liquid tank 31.

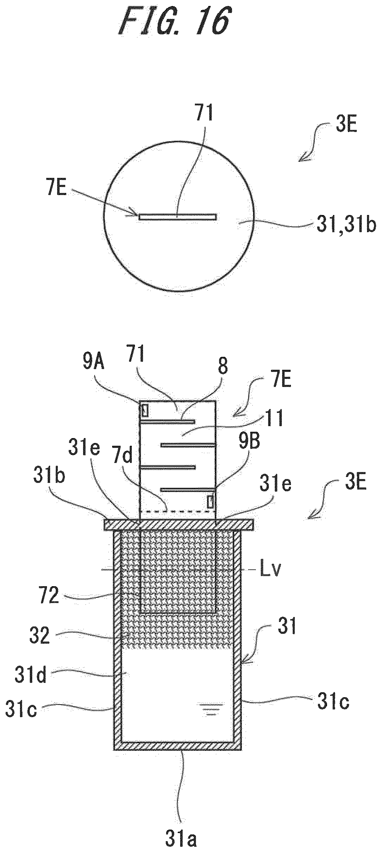

Fig. 16 is a view illustrating a cartridge 3E according to a first modification of the fifth embodiment. The porous heat-generating sheet 7E provided to the cartridge 3E has a structure identical to that of the porous heat-generating sheet 7D illustrated in Fig. 15, except for a feature in which one suctioning unit 72 is connected to the lower side 7d of the heater unit 71. The porous heat-generating sheet 7E entirely has a flat plate-like shape. The porous heat-generating sheet 7E entirely has a flat plate-like shape, and the suctioning unit 72 is inserted in the liquid reserving space 31d through the insertion hole 31e formed in the lid 31b of the liquid tank 31. That is, in the cartridge 3D, the porous heat-generating sheet 7D is set with respect to the liquid tank 31 while the heater unit 71 of the flat plate-like porous heat-generating sheet 7D is exposed to the outside of the liquid tank 31 and the suctioning unit 72 is inserted outside the liquid tank 31.

Fig. 17 is a view illustrating a porous heat-generating sheet 7F according to a second modification of the fifth embodiment. In the porous heat-generating sheet 7F, one suctioning unit 72 is connected to the right side 7b of the heater unit 71. The porous heat-generating sheet 7F is rounded into a cylindrical shape. In the example illustrated in Fig. 17, an insulation member 73 is provided between the upper side 7c and the lower side 7d of the heater unit 71. The upper side 7c and the lower side 7d of the heater unit 71 are insulated from each other by the insulation member 73. In Fig. 17, illustration of the slits 8, the positive electrode 9A, the negative electrode 9B, and the like, in the heater unit 71 is omitted. Also, in the porous heat-generating sheet 7F, instead of interposing of the insulation member 73 between the upper side 7c and the lower side 7d of the heater unit 71, the porous heat-generating sheet 7F may be rounded into a C-like shape such that a gap is formed between the upper side 7c and the lower side 7d.

### <Sixth embodiment>

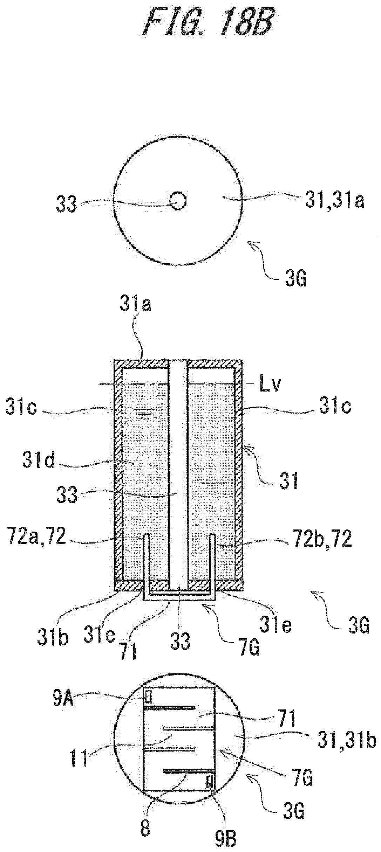

Fig. 18A is a view illustrating an electric cigarette 1G according to the sixth embodiment. Fig. 18B is a view illustrating a cartridge 3G according to the sixth embodiment. The cartridge 3G has the porous heat-generating sheet 7 illustrated in Fig. 4. The liquid tank 31 in the cartridge 3G has an annular shape, and a hollow through passage 33 is provided in the center portion thereof. As illustrated in Fig. 18A, the hollow through passage 33 in the liquid tank 31 of the cartridge 3G penetrates the liquid tank 31 in the up/down direction. As in the first embodiment, the suctioning units 72 are inserted in the liquid reserving space 31d through the insertion hole 31e provided in the lid 31b of the liquid tank 31 so that the porous heat-generating sheet 7 is in contact with the aerosol-generating liquid.

In the cartridge 3G, the lid 31b of the liquid tank 31 is accommodated in the accommodating cavity 23 so as to face the deep side (inner side) of the accommodating cavity 23. That is, in the cartridge 3G according to the sixth embodiment, the lid 31b is accommodated in the accommodating cavity 23 such that the up/down direction thereof is opposite to that in the cartridge 3 according to the first embodiment. That is, in the cartridge 3G, the bottom 31a side of the liquid tank 31 is arranged so as to face the mouthpiece unit 4. In the electric cigarette 1G, the air intake port 43 is provided in the body-side housing 20 of the body unit 2. Together with aerosols generated by a porous heat-generating sheet 7, air taken in the body-side housing 20 from the outside through the air intake port 43 passes through the hollow through passage 33 and the inner passage 45 in the mouthpiece unit 4, and reaches the inhalation port 42. A user can inhale the aerosols from the inhalation port 42.

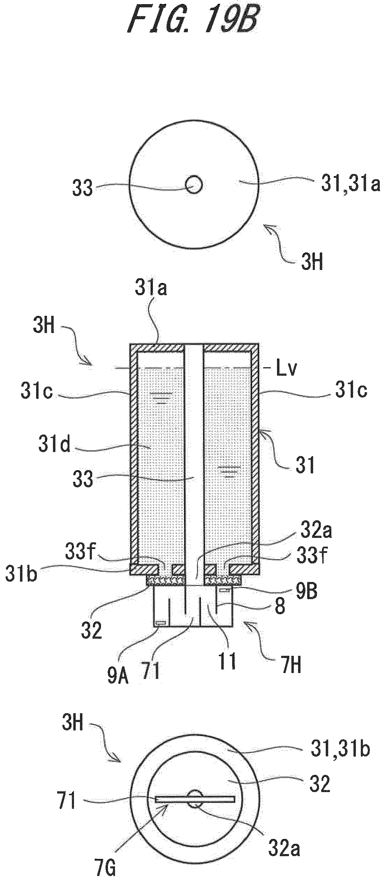

Fig. 19A is a view illustrating an electric cigarette 1H according to a modification of the sixth embodiment. Fig. 19B is a view illustrating a cartridge 3H according to the modification of the sixth embodiment. Also in the cartridge 3H, the liquid tank 31 has an annular shape in which the hollow through passage 33 is provided on the center side thereof, as in the cartridge 3G. The liquid supply member 32 made of cotton fibers, for example, is disposed on the outer surface side of the lid 31b of the liquid tank 31 in the cartridge 3H. The liquid supply member 32 has a disc shape, and has a vent hole 32a at a position corresponding to the hollow through passage 33 in the liquid tank 31. A liquid supply hole 33f for supplying the aerosol-generating liquid stored in the liquid tank 31 (liquid reserving space 31d) to the liquid supply member 32 is provided in the lid 31b of the liquid tank 31.

The cartridge 3G of the present embodiment has the porous heat-generating sheet 7H formed of only the heater unit 71 having the same structure as that of the heater unit 71 of the porous heat-generating sheet 7B according to the third embodiment. In the example illustrated in Fig. 19B, in a state where an end surface of the porous heat-generating sheet 7H abuts on an outer surface of the liquid supply member 32, the porous heat-generating sheet 7H is fixed to the liquid supply member 32. In the electric cigarette 1H thus configured, the aerosol-generating liquid stored in the liquid tank 31 (liquid reserving space 31d) of the cartridge 3H is supplied to the porous heat-generating sheet 7H (heater unit 71) via the liquid supply member 32, and is held in the heater unit 71. When a current flow is caused between the electrodes on the heater unit 71, the aerosol-generating liquid held in the heater unit 71 is atomized, whereby aerosols are generated.

As illustrated in Fig. 19A, in the electric cigarette 1H, the air intake port 43 is provided in the body-side housing 20 of the body unit 2. Together with aerosols generated by the porous heat-generating sheet 7H (heater unit 71), air taken in the body-side housing 20 from the outside through the air intake port 43 passes through the vent hole 32a in the liquid supply member 32, the hollow through passage 33 in the liquid tank 31, and the inner passage 45 in the mouthpiece unit 4, and reaches the inhalation port 42. A user can inhale the aerosols from the inhalation port 42.

The preferred embodiments of the present invention have been described above. However, it is obvious to a person skilled in the art that various changes, modifications, or combinations thereof can be made in the aerosol inhaler, the cartridge applied to the aerosol inhaler, and the porous heat-generating sheet according to the present invention.

### Reference Signs List

- 1 ...: electric cigarette
- 2 ...: body unit
- 21 ...: battery
- 22 ...: electronic control unit
- 24 ...: accommodating cavity
- 3 ...: cartridge
- 31 ...: liquid tank
- 32 ...: liquid supply member
- 4 ...: mouthpiece unit
- 42 ...: inhalation port
- 5 ...: hinge
- 7 ...: porous heat-generating sheet
- 71 ...: heater unit
- 72 ...: suctioning unit
- 8 ...: slit
- 9A ...: positive electrode
- 9B ...: negative electrode
- 10 ...: electric path
- 11 ...: meandering electric path unit
- 110 ...: linear electric path portion
- 120 ...: bent electric path portion
- 12 ...: positive electrode-provided electric path unit
- 13 ...: negative electrode-provided electric path unit

## Claims

1. A cartridge (3) for aerosol inhaler (1) comprising:
a liquid reservoir (31) that stores an aerosol-generating liquid; and
a heat-generating sheet (7) that is provided with a positive electrode (9A) and a negative electrode (9B), and that atomizes the aerosol-generating liquid supplied from the liquid reservoir (31), by generating heat when a current flow is caused between the positive electrode (9A) and the negative electrode (9B), wherein
the heat-generating sheet (7) is formed of a porous material, and an electric path connecting the positive electrode (9A) and the negative electrode (9B) includes a meandering electric path unit (11) that is formed into a meandering shape by a plurality of slits (8) linearly provided in the heat-generating sheet (7),
the meandering electric path unit (11) is formed by sequentially connecting a linear electric path portion (110) having a linear shape and a bent electric path portion (120) obtained by bending the linear electric path portion (110),
the positive electrode (9A) is provided on a positive electrode-provided electric path unit (12) that is connected to one end of the meandering electric path unit (11), and the negative electrode (9B) is provided on a negative electrode-provided electric path unit (13) that is connected to the other end of the meandering electric path unit (11),
the slits (8) separating the linear electric path portions (110) from one another are arranged in parallel at a fixed interval in the heat-generating sheet (7)
the linear electric path portions (110) are separated from one another by an overlapping section where the adjacent slits (8) overlap each other in an extension direction thereof, **characterised in that**
a length dimension of the overlapping section is set to be equal to or larger than a distance dimension between the adjacent slits (8) or the total length dimension of the overlapping section is set to be equal to or larger than a dimension of a straight line connecting the positive electrode (9A) and the negative electrode (9B).

2. The cartridge (3) for aerosol inhaler (1) according to claim 1, wherein
in the heat-generating sheet (7), an electrode effective width, of the positive electrode (9A), in a direction orthogonal to a direction in which current flows out from the positive electrode (9A) is relatively narrower than the electrode path width of a minimum electric path width portion, of an electric path connecting the positive electrode (9A) and the negative electrode (9B), where the electric path width becomes narrowest.

3. The cartridge (3) for aerosol inhaler (1) according to any one of claims 1 to 2, wherein
the slits (8) extend in an extension direction of the linear electric path portion (110).

4. The cartridge (3) for aerosol inhaler (1) according to claim 2, wherein
in the heat-generating sheet (7), the positive electrode (9A) is formed by being extended, within a plane of the heat-generating sheet (7), in a direction orthogonal to a direction of the electrode effective width, and a band-like virtual band region having a width equal to the electrode effective width does not include an end of the slit (8) that extends from an edge of the heat-generating sheet (7) toward an inner side on the plane of the heat-generating sheet (7).

5. An aerosol inhaler (1) comprising the cartridge (3) for aerosol inhaler (1) according to any one of claims 1 to 4.

6. A heat-generating sheet (7) for aerosol inhaler (1) that is provided with a positive electrode (9A) and a negative electrode (9B), and that atomizes an aerosol-generating liquid supplied thereto from a liquid reservoir (31) of the aerosol inhaler (1), by generating heat when a current flow is caused between the positive electrode (9A) and the negative electrode (9B), wherein
the heat-generating sheet (7) is formed of a porous material, and an electric path connecting the positive electrode (9A) and the negative electrode (9B) includes a meandering electric path unit (11) that is formed into a meandering shape by a plurality of slits (8) linearly provided in the heat-generating sheet (7),
the meandering electric path unit (11) is formed by sequentially connecting a linear electric path portion (110) having a linear shape and a bent electric path portion (120) obtained by bending the linear electric path portion (110),
the positive electrode (9A) is provided on a positive electrode-provided electric path unit (12) that is connected to one end of the meandering electric path unit (11), and the negative electrode (9B) is provided on a negative electrode-provided electric path unit (13) that is connected to the other end of the meandering electric path unit (11),
the slits (8) separating the linear electric path portions (110) from one another are arranged in parallel at a fixed interval in the heat-generating sheet (7)
the linear electric path portions (110) are separated from one another by an overlapping section where the adjacent slits (8) overlap each other in an extension direction thereof, **characterised in that**
a length dimension of the overlapping section is set to be equal to or larger than a distance dimension between the adjacent slits (8) or the total length dimension of the overlapping section is set to be equal to or larger than a dimension of a straight line connecting the positive electrode (9A) and the negative electrode (9B).

## Patentansprüche

1. Patrone (3) für einen Aerosol-Inhalator (1), umfassend:
einen Flüssigkeitsbehälter (31), der eine aerosolerzeugende Flüssigkeit speichert; und
eine wärmeerzeugende Platte (7), die mit einer positiven Elektrode (9A) und einer negativen Elektrode (9B) bereitgestellt ist und die die aerosolerzeugende Flüssigkeit, die von dem Flüssigkeitsbehälter (31) zugeführt wird, zerstäubt, indem sie Wärme erzeugt, wenn ein Stromfluss zwischen der positiven Elektrode (9A) und der negativen Elektrode (9B) bewirkt wird, wobei
die wärmeerzeugende Platte (7) aus einem porösen Material gebildet ist und ein elektrischer Pfad, der die positive Elektrode (9A) und die negative Elektrode (9B) verbindet, eine mäandernde elektrische Pfadeinheit (11) einschließt, die durch eine Vielzahl von Schlitzen (8), die linear in der wärmeerzeugenden Platte (7) bereitgestellt sind, in eine mäandernde Form gebracht wird,
die mäandernde elektrische Pfadeinheit (11) durch aufeinanderfolgendes Verbinden eines linearen elektrischen Pfadabschnitts (110), der eine lineare Form aufweist, und eines gebogenen elektrischen Pfadabschnitts (120), der durch Biegen des linearen elektrischen Pfadabschnitts (110) erhalten wird, gebildet wird,
die positive Elektrode (9A) an einer mit einer positiven Elektrode bereitgestellten elektrischen Pfadeinheit (12) bereitgestellt ist, die mit einem Ende der mäandernden elektrischen Pfadeinheit (11) verbunden ist, und die negative Elektrode (9B) an einer mit einer negativen Elektrode bereitgestellten elektrischen Pfadeinheit (13) bereitgestellt ist, die mit dem anderen Ende der mäandernden elektrischen Pfadeinheit (11) verbunden ist,
die Schlitze (8), die die linearen elektrischen Pfadabschnitte (110) voneinander trennen, in einem festen Abstand in der wärmeerzeugenden Platte (7) parallel angeordnet sind
die linearen elektrischen Pfadabschnitte (110) durch einen überlappenden Bereich voneinander getrennt sind, in dem die benachbarten Schlitze (8) einander in einer Erstreckungsrichtung davon überlappen, **dadurch gekennzeichnet, dass**
eine Längenabmessung des überlappenden Bereichs so eingestellt ist, dass sie gleich oder größer als eine Abstandsabmessung zwischen den benachbarten Schlitzen (8) ist, oder die Gesamtlängenabmessung des überlappenden Bereichs so eingestellt ist, dass sie gleich oder größer als eine Abmessung einer geraden Linie ist, die die positive Elektrode (9A) und die negative Elektrode (9B) verbindet.

2. Patrone (3) für einen Aerosol-Inhalator (1) nach Anspruch 1, wobei
in der wärmeerzeugenden Platte (7) eine effektive Elektrodenbreite der positiven Elektrode (9A) in einer Richtung orthogonal zu einer Richtung, in der Strom von der positiven Elektrode (9A) herausfließt, relativ schmaler ist als die Elektrodenpfadbreite eines minimalen elektrischen Pfadbreitenteils eines elektrischen Pfades, der die positive Elektrode (9A) und die negative Elektrode (9B) verbindet, wo die elektrische Pfadbreite am schmalsten wird.

3. Patrone (3) für Aerosol-Inhalator (1) nach einem der Ansprüche 1 bis 2, wobei
sich die Schlitze (8) in einer Erstreckungsrichtung des linearen elektrischen Pfadabschnitts (110) erstrecken.

4. Patrone (3) für einen Aerosol-Inhalator (1) nach Anspruch 2, wobei
in der wärmeerzeugenden Platte (7) die positive Elektrode (9A) dadurch gebildet wird, dass sie sich innerhalb einer Ebene der wärmeerzeugenden Folie (7) in einer Richtung orthogonal zu einer Richtung der effektiven Elektrodenbreite erstreckt, und ein bandartiger virtueller Bandbereich mit einer Breite, die gleich der effektiven Elektrodenbreite ist, kein Ende des Schlitzes (8) einschließt, der sich von einer Kante der wärmeerzeugenden Platte (7) zu einer Innenseite in der Ebene der wärmeerzeugenden Platte (7) erstreckt.

5. Aerosol-Inhalator (1), umfassend die Patrone (3) für Aerosol-Inhalator (1) nach einem der Ansprüche 1 bis 4.

6. Wärmeerzeugende Platte (7) für einen Aerosol-Inhalator (1), die mit einer positiven Elektrode (9A) und einer negativen Elektrode (9B) bereitgestellt ist und die eine ihr aus einem Flüssigkeitsbehälter (31) des Aerosol-Inhalators (1) zugeführte aerosolerzeugende Flüssigkeit zerstäubt, indem sie Wärme erzeugt, wenn ein Stromfluss zwischen der positiven Elektrode (9A) und der negativen Elektrode (9B) bewirkt wird, wobei
die wärmeerzeugende Platte (7) aus einem porösen Material gebildet ist und ein elektrischer Pfad, der die positive Elektrode (9A) und die negative Elektrode (9B) verbindet, eine mäandernde elektrische Pfadeinheit (11) einschließt, die durch eine Vielzahl von Schlitzen (8), die linear in der wärmeerzeugenden Platte (7) bereitgestellt sind, in eine mäandernde Form gebracht wird,
die mäandernde elektrische Pfadeinheit (11) durch aufeinanderfolgendes Verbinden eines linearen elektrischen Pfadabschnitts (110), der eine lineare Form aufweist, und eines gebogenen elektrischen Pfadabschnitts (120), der durch Biegen des linearen elektrischen Pfadabschnitts (110) erhalten wird, gebildet wird,
die positive Elektrode (9A) an einer mit einer positiven Elektrode bereitgestellten elektrischen Pfadeinheit (12) bereitgestellt ist, die mit einem Ende der mäandernden elektrischen Pfadeinheit (11) verbunden ist, und die negative Elektrode (9B) an einer mit einer negativen Elektrode bereitgestellten elektrischen Pfadeinheit (13) bereitgestellt ist, die mit dem anderen Ende der mäandernden elektrischen Pfadeinheit (11) verbunden ist,
die Schlitze (8), die die linearen elektrischen Pfadabschnitte (110) voneinander trennen, in einem festen Abstand in der wärmeerzeugenden Platte (7) parallel angeordnet sind
die linearen elektrischen Pfadabschnitte (110) durch einen überlappenden Bereich voneinander getrennt sind, in dem die benachbarten Schlitze (8) einander in einer Erstreckungsrichtung davon überlappen, **dadurch gekennzeichnet, dass**
eine Längenabmessung des überlappenden Bereichs so eingestellt ist, dass sie gleich oder größer als eine Abstandsabmessung zwischen den benachbarten Schlitzen (8) ist, oder die Gesamtlängenabmessung des überlappenden Bereichs so eingestellt ist, dass sie gleich oder größer als eine Abmessung einer geraden Linie ist, die die positive Elektrode (9A) und die negative Elektrode (9B) verbindet.

## Revendications

1. Cartouche (3) pour inhalateur d'aérosol (1) comprenant :
un réservoir de liquide (31) qui stocke un liquide générateur d'aérosol ; et
une feuille génératrice de chaleur (7) qui est fournie avec une électrode positive (9A) et une électrode négative (9B), et qui atomise le liquide générateur d'aérosol alimenté à partir du réservoir de liquide (31), en générant de la chaleur lorsqu'un flux de courant est provoqué entre l'électrode positive (9A) et l'électrode négative (9B), dans laquelle
la feuille génératrice de chaleur (7) est formée d'un matériau poreux, et un trajet électrique reliant l'électrode positive (9A) à l'électrode négative (9B) inclut une unité de trajet électrique sinueuse (11) qui est formée en une forme sinueuse par une pluralité de fentes (8) fournies linéairement dans la feuille génératrice de chaleur (7),
l'unité de trajet électrique sinueuse (11) est formée en reliant séquentiellement une partie de trajet électrique linéaire (110) présentant une forme linéaire et une partie de trajet électrique courbée (120) obtenue en courbant la partie de trajet électrique linéaire (110),
l'électrode positive (9A) est fournie sur une unité de trajet électrique fournie à électrode positive (12) qui est reliée à une extrémité de l'unité de trajet électrique sinueuse (11), et l'électrode négative (9B) est fournie sur une unité de trajet électrique fournie à électrode négative (13) qui est reliée à l'autre extrémité de l'unité de trajet électrique sinueuse (11),
les fentes (8) séparant les parties de trajet électrique linéaires (110) les unes des autres sont agencées parallèlement à un intervalle fixe dans la feuille génératrice de chaleur (7)
les parties de trajet électrique linéaires (110) sont séparées les unes des autres par une section de chevauchement où les fentes adjacentes (8) se chevauchent les unes les autres dans une direction d'extension de celles-ci, **caractérisée en ce que**
une dimension longitudinale de la section de chevauchement est fixée de manière à être supérieure ou égale à une dimension de distance entre les fentes adjacentes (8) ou la dimension de la longueur totale de la section de chevauchement est fixée de manière à être supérieure ou égale à une dimension d'une ligne droite reliant l'électrode positive (9A) à l'électrode négative (9B).

2. Cartouche (3) pour inhalateur d'aérosol (1) selon la revendication 1, dans laquelle
dans la feuille génératrice de chaleur (7), une largeur réelle de l'électrode, de l'électrode positive (9A), dans une direction orthogonale à une direction dans laquelle du courant circule hors de l'électrode positive (9A) est relativement plus étroite que la largeur du trajet de l'électrode d'une partie de la largeur du trajet électrique minimale, d'un trajet électrique reliant l'électrode positive (9A) à l'électrode négative (9B), où la largeur du trajet électrique devient plus étroit.

3. Cartouche (3) pour inhalateur d'aérosol (1) selon l'une quelconque des revendications 1 à 2, dans laquelle
les fentes (8) s'étendent dans une direction d'extension de la partie de trajet électrique linaire (110).

4. Cartouche (3) pour inhalateur d'aérosol (1) selon la revendication 2, dans laquelle
dans la feuille génératrice de chaleur (7), l'électrode positive (9A) est formée en étant étendue, à l'intérieur d'un plan de la feuille génératrice de chaleur (7), dans une direction orthogonale à une direction de la largeur réelle de l'électrode, et une région de bande virtuelle en forme de bande présentant une largeur égale à la largeur réelle de l'électrode n'inclut pas une extrémité de la fente (8) qui s'étend d'un bord de la feuille génératrice de chaleur (7) vers un côté intérieur sur le plan de la feuille génératrice de chaleur (7).

5. Inhalateur d'aérosol (1) comprenant la cartouche (3) de l'inhalateur d'aérosol (1) selon l'une quelconque des revendications 1 à 4.

6. Feuille génératrice de chaleur (7) pour inhalateur d'aérosol (1) qui est fournie avec une électrode positive (9A) et une électrode négative (9B), et qui atomise le liquide générateur d'aérosol alimenté à celle-ci par un réservoir de liquide (31) de l'inhalateur d'aérosol (1), en générant de la chaleur lorsqu'un flux de courant est provoqué entre l'électrode positive (9A) et l'électrode négative (9B), dans laquelle
la feuille génératrice de chaleur (7) est formée d'un matériau poreux, et un trajet électrique reliant l'électrode positive (9A) à l'électrode négative (9B) inclut une unité de trajet électrique sinueuse (11) qui est formée en une forme sinueuse par une pluralité de fentes (8) fournies linéairement dans la feuille génératrice de chaleur (7),
l'unité de trajet électrique sinueuse (11) est formée en reliant séquentiellement une partie de trajet électrique linéaire (110) présentant une forme linéaire et une partie de trajet électrique courbée (120) obtenue en courbant la partie de trajet électrique linéaire (110),
l'électrode positive (9A) est fournie sur une unité de trajet électrique fournie à électrode positive (12) qui est reliée à une extrémité de l'unité de trajet électrique sinueuse (11), et l'électrode négative (9B) est fournie sur une unité de trajet électrique fournie à électrode négative (13) qui est reliée à l'autre extrémité de l'unité de trajet électrique sinueuse (11),
les fentes (8) séparant les parties de trajet électrique linéaires (110) les unes des autres sont agencées parallèlement à un intervalle fixe dans la feuille génératrice de chaleur (7)
les parties de trajet électrique linéaires (110) sont séparées les unes des autres par une section de chevauchement où les fentes adjacentes (8) se chevauchent les unes les autres dans une direction d'extension de celles-ci, **caractérisée en ce que**
une dimension longitudinale de la section de chevauchement est fixée de manière à être supérieure ou égale à une dimension de distance entre les fentes adjacentes (8) ou la dimension de la longueur totale de la section de chevauchement est fixée de manière à être supérieure ou égale à une dimension d'une ligne droite reliant l'électrode positive (9A) à l'électrode négative (9B).
